(19) Europäisches Patentamt
European Patent Office
Office européen des brevets

(11) **EP 2 189 120 A1**

(12) **DEMANDE DE BREVET EUROPEEN**

(43) Date de publication:
**26.05.2010 Bulletin 2010/21**

(51) Int Cl.:
***A61B 17/06*** *(2006.01)*     *A61B 17/00* *(2006.01)*
***A61B 18/00*** *(2006.01)*

(21) Numéro de dépôt: **08169414.3**

(22) Date de dépôt: **19.11.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(71) Demandeur: **Université Libre de Bruxelles**
**1050 Bruxelles (BE)**

(72) Inventeurs:
• **Deviere, Jacques**
**1470, Genappe (BE)**

• **Cauche, Nicolas**
**1160, Bruxelles (BE)**
• **Delchambre, Alain**
**1170, Bruxelles (BE)**
• **Dugardeyn, Sonia**
**7090, Braine le Comte (BE)**

(74) Mandataire: **Van Malderen, Joëlle et al**
**pronovem - Office Van Malderen**
**Avenue Josse Goffin 158**
**1082 Bruxelles (BE)**

(54) **Aiguille chirurgicale à mémoire de forme**

(57)     La présente invention concerne une aiguille chirurgicale à mémoire de forme comprenant un corps d'aiguille ayant deux extrémités (1,2), distale et proximale, ledit corps présentant au moins deux formes stables différentes, soit une première forme stable à une première gamme de température et une deuxième forme stable à une deuxième gamme de température, la première gamme de température étant strictement inférieure à la deuxième gamme de température.

Fig. 1

EP 2 189 120 A1

## Description

### Domaine d'application

La présente invention concerne le domaine de la suture d'une muqueuse située à l'intérieur du corps humain.

Particulièrement la présente invention se réfère à une aiguille chirurgicale et en particulier à une aiguille endoscopique dont l'accès au site à traiter s'effectuera par voie endoscopique.

La présente invention se rapporte également au dispositif permettant d'utiliser ladite aiguille chirurgicale.

Enfin, la présente invention se rapporte à un traitement thérapeutique permettant d'utiliser de telles aiguilles chirurgicales.

### Problème technique

Lors d'une opération chirurgicale, lorsqu'il faut effectuer une suture en bourse, en surjet, une plicature, ou une anastomose, le chirurgien utilise une aiguille courbe rigide et affûtée. La forme courbe de cette aiguille lui permet par un mouvement de rotation de passer l'aiguille de l'autre coté de la paroi et de la faire revenir sans devoir plier cette paroi. La suture ne peut être réalisée qu'à condition qu'une aiguille courbe puisse être amenée sur le site à traiter et que l'aiguille puisse être manipulée avec les degrés de liberté nécessaires à la réalisation de la suture.

Dans certaines interventions d'endoscopie souple telles que par exemple le traitement d'une hémorragie gastrique importante nécessitant une suture au sein de la cavité gastrique, ces deux conditions ne sont pas remplies. Le gastro-entérologue ne peut pas actuellement amener une aiguille courbe vers la cavité gastrique car les voies d'accès naturelles à la cavité gastrique sont trop étroites. De ce fait, lorsqu'une suture doit être effectuée au sein de la cavité gastrique, le patient doit subir une intervention chirurgicale lourde qui doit permettre une ouverture de tous les tissus pour passer à travers la surface du corps et atteindre l'organe en question, ce qui augmente considérablement le traumatisme, et ce spécialement pour les patients obèses ou les grands brûlés dont la cicatrisation de la peau est délicate. Cela représente une des principales limitations de l'endoscopie thérapeutique souple qui se pratique par les voies naturelles.

Plusieurs solutions pour réaliser les sutures ont été proposées et peuvent être classifiées de la façon suivante.

Une catégorie de sutures s'effectue en réalisant un pli - par exemple par sussion - et en passant une aiguille droite dans le pli. Par exemple, ces interventions sont utilisées pour former des plicatures situées à des endroits précis de la cavité gastrique ou du bas oesophage.

Spécifiquement, pour le traitement du reflux gastro-oesophagien, plusieurs dispositifs permettent de réaliser une plicature du cardia (NDO®, Plicator®; Bard Therapeutic®, Endocinch®) ou de placer un manchon autour du cardia. Ces techniques utilisent un matériel sophistiqué, soit attaché au bout de l'endoscope, soit consistant en un instrument complexe dans lequel est introduit un endoscope. Le but de ces dispositifs est de palier le fait que l'endoscopiste est un chirurgien à une seule main qui ne peut tirer sur le tissu pour faire une suture. Ces dispositifs visant à réaliser une plicature du cardia permettent de saisir la totalité de la paroi gastrique et de la suturer à une autre partie de cette paroi.

Pour la création d'anastomoses (gastro-entérostomie) entre l'estomac et l'intestin grêle, une suture des deux parois (séro-séreuse) est nécessaire. Certaines techniques sont actuellement développées (T Tags, T bars) mais ne permettent que des sutures ponctuelles et sont associées au risque de ponctionner des organes adjacents lorsque l'on utilise des aiguilles droites et rigides. La réalisation d'une suture en surjet n'est pas possible.

Plusieurs documents de l'état de la technique décrivent des dispositifs permettant d'amener une aiguille courbe à l'intérieur du corps d'un patient. En particulier, les documents W09508296 et EP0529675 décrivent une aiguille qui peut effectuer une transition entre deux formes stables à deux gammes de températures différentes.

Dans le document W09508296, l'aiguille est réalisée dans un matériau à mémoire de forme. La forme de l'aiguille endoscopique est modifiée par la température environnante de sorte qu'à température ambiante (définie dans ce document comme étant entre 0°C et 24°C), l'aiguille est droite et qu'à une température comprise entre 25°C et 40°C, l'aiguille devient courbe.

Dans le document EP0529675, l'aiguille est réalisée en alliage à mémoire de forme. La forme de l'aiguille endoscopique est modifiée par des sources de chaleur extérieure telles que "illumination light", "laser" ou "cautery" de sorte que l'aiguille est droite à une température inférieure à 25°C et que l'aiguille est courbe à une température supérieure à 35°C.

Les aiguilles décrites dans les deux documents cités ci-dessus ne sont pas aussi rigides que les aiguilles courbes habituellement utilisées par les chirurgiens. En effet, dans les exemples cités dans ces deux documents, les aiguilles dans leur forme courbe sont retirées du corps du patient en appliquant une contrainte mécanique par l'intermédiaire d'un tube.

Pour que les aiguilles décrites dans les deux documents cités ci-dessus changent de forme, elles doivent être exposées à une source de chaleur sur une grande partie de leurs surfaces extérieures. Ces aiguilles ne peuvent être utilisées que sous leur forme courbe dans le corps. Ceci limite les possibilités thérapeutiques par rapport à une aiguille qui pourrait être utilisée à la fois sous sa forme courbe et sous sa forme rectiligne à l'intérieur du corps humains.

Le passage d'une forme courbe à une forme droite à l'intérieur du corps humain permettrait d'une part d'augmenter les possibilités de suture et d'autre part d'utiliser une aiguille de rigidité équivalente à celle qu'offre par exemple les aiguilles du type "Ethilon® CPX 1 (45 mm)" fournie par Ethicon® (Johnson&Johnson®).

## Principaux éléments caractéristiques

La présente invention concerne une aiguille chirurgicale à mémoire de forme comprenant un corps d'aiguille ayant deux extrémités, distale et proximale, ledit corps présentant au moins deux formes stables différentes, soit une première forme stable à une première gamme de température et une deuxième forme stable à une deuxième gamme de température, la première gamme de température étant strictement inférieure à la deuxième gamme de température ; caractérisée en ce que le matériau constituant le corps de l'aiguille comprend au moins un polymère à mémoire de forme et au moins un matériau conducteur thermique et/ou électrique ; la totalité du corps de l'aiguille étant apte à être chauffée en appliquant une source de chaleur et/ou de courant. La variation de température induite par la source de chaleur et/ou de courant dans l'aiguille permet le passage de la première forme stable à la deuxième forme stable.

Par forme stable, on entend une forme géométrique qui reste stable essentiellement pour des températures appartenant à une gamme de température. Avantageusement, cette aiguille ne subira pas de modification de forme ou de structure si on y applique des forces mécaniques.

De préférence, la première forme stable peut-être une forme rectiligne, tandis que la deuxième forme stable peut être une forme courbée de l'aiguille.

De préférence, la totalité du corps de l'aiguille est également apte à être refroidie en y appliquant une source de froid.

De préférence, la variation de température induite par la source de froid dans l'aiguille permet le passage de la deuxième forme stable à une deuxième gamme de température à une troisième forme stable à une troisième gamme de température. Avantageusement, cette troisième forme stable correspond à la première forme stable.

De préférence, ladite source de chaleur et/ou de courant est en contact direct avec une des extrémités, de préférence l'extrémité proximale de l'aiguille.

De préférence, ladite source de froid est en contact direct avec l'une des extrémités, de préférence l'extrémité proximale de l'aiguille.

Préférentiellement, au moins une des extrémités, et de préférence l'extrémité proximale de ladite aiguille est réalisée en un matériau conducteur thermique et/ou électrique qui peut être différent ou identique du matériau conducteur thermique et/ou électrique constituant le corps de l'aiguille. Ladite extrémité proximale est de préférence en contact direct avec ledit matériau conducteur contenu dans le reste de l'aiguille.

De préférence, la forme de l'aiguille à la première gamme de température est rectiligne.

De préférence, la forme de l'aiguille à la deuxième gamme de température est courbe.

De préférence, la forme de l'aiguille à la troisième gamme de température est à nouveau rectiligne.

Avantageusement la transition entre la première et la deuxième formes stables s'effectue à une première température de transition.

Avantageusement, la transition entre la deuxième et la troisième formes stables s'effectue à une deuxième température de transition. De manière particulièrement avantageuse, la première température de transition correspond à la deuxième température de transition.

Avantageusement, la première gamme de température est strictement inférieure à la première température de transition.

Avantageusement, la troisième gamme de température est strictement inférieure à la deuxième température de transition.

Avantageusement, la deuxième gamme de température est strictement supérieure à la première et à la deuxième température de transition.

Avantageusement, la transition entre lesdites première et deuxième formes s'effectue à une température de transition comprise entre une température minimum correspondant à la température corporelle soit environ 37°C et une température de environ 50°C, de préférence à une température de transition comprise entre 40°C et 45°C.

Avantageusement, la transition entre les deuxième et troisième formes d'exécution s'effectue à une température de transition comprise entre une température minimum correspondant à la température corporelle soit environ 37°C et une température de environ 50°C et de préférence entre 40°C et 45°C.

De préférence, en-dessous de sa ou ses température(s) de transition, l'aiguille est rectiligne.

De préférence, au-dessus de sa ou ses températures de transition, l'aiguille est courbe.

Préférentiellement, lorsque la température est strictement inférieure à la température corporelle (37°C) l'aiguille se retrouve dans sa forme rectiligne.

Préférentiellement, lorsque la température est strictement supérieure à 45°C l'aiguille se retrouve dans la forme courbe.

Préférentiellement, l'extrémité distale de l'aiguille est affûtée.

Préférentiellement, l'extrémité proximale de l'aiguille accueille du fil chirurgical.

## Description détaillée de plusieurs formes d'exécutions préférées de l'invention

La présente invention est une aiguille en matériau de préférence composite contenant des éléments conducteurs - mélange de polymères à mémoire de forme et de particules et/ou fibres conductrices électriques et/ou thermiques - possédant (au moins) deux formes stables.

La première forme de travail est une aiguille rectiligne.

La deuxième forme de travail est une aiguille comprenant obligatoirement une partie courbe et optionnellement une partie droite.

La partie courbe est rigide, affûtée et sa forme générale est par exemple semblable à un arc de cercle.

L'angle formé par les deux rayons joignant les deux extrémités de la partie courbe est compris entre environ 120° et environ 200°.

Cette forme peut être équivalente à l'aiguille du type "Ethilon® CPX 1 (45 mm)" fournie par Ethicon® (Johnson&Johnson®).

Préférentiellement, le rayon de la courbure de l'aiguille courbe est compris entre environ 2.5 mm et environ 22.5 mm.

Préférentiellement, une extrémité de l'aiguille est affûtée de manière à pénétrer facilement le tissu.

Préférentiellement, un fil de suture chirurgical peut être attaché à l'autre extrémité de l'aiguille.

La rigidité en flexion d'une aiguille peut être caractérisée en multipliant le module de Young (E) du matériau de l'aiguille par le moment d'inertie (I) de la section droite de l'aiguille par rapport à l'axe neutre. De ce fait, afin d'obtenir une rigidité flexionnelle équivalente aux aiguilles classiques par exemple de type "Ethilon® CPX 1 (45 mm)" fournie par Ethicon® (Johnson&Johnson®), le produit E I doit être conservé. On doit donc vérifier que :

$$E_a\ I_a\ =\ E_b\ I_b$$

$E_a$ : Module de Young d'une aiguille classiquement utilisée en chirurgie.

$I_a$ : Moment d'inertie de la section droite d'une aiguille, classiquement utilisée en chirurgie, par rapport à l'axe neutre.

$E_b$ : Module de Young de l'aiguille décrite dans la présente invention.

$I_b$ : Moment d'inertie de la section droite de l'aiguille décrite dans la présente invention par rapport à l'axe neutre.

Préférentiellement, le diamètre du cercle circonscrit à la section de l'aiguille ne dépassera pas environ 4 mm.

De manière encore plus préférentielle, le diamètre du cercle circonscrit à la section de l'aiguille ne dépassera pas environ 3 mm.

Le matériau constitutif de l'aiguille est un matériau composite à mémoire de forme pouvant comprendre n'importe quel type de polymères et n'importe quel type de particules et/ou fibres conductrices électriques et/ou thermiques.

La surface d'une extrémité de l'aiguille est conductrice électriquement et/ou thermiquement de manière à apporter (ou retirer) rapidement de l'énergie calorifique à l'aiguille en permettant un changement de température par conduction à l'aiguille entière.

Ladite surface est connectée aux particules et/ou fibres conductrices électriques et/ou thermiques constituant l'aiguille.

Préférentiellement, la surface de l'autre extrémité de l'aiguille peut être conductrice de manière à apporter (ou retirer) rapidement de l'énergie calorifique à l'aiguille en permettant un changement de température par conduction à l'aiguille entière.

Ladite surface est connectée aux particules et/ou fibres conductrices électriques et/ou thermiques constituant l'aiguille.

Le passage de l'aiguille droite à l'aiguille courbe se fait lorsque la température de l'aiguille atteint une première température de transition $Ts_1$ qui est comprise entre environ 37 et environ 50 degrés Celsius.

Inversément, le passage de l'aiguille courbe à l'aiguille droite se fait lorsque la température de l'aiguille atteint une deuxième température de transition $Ts_2$ qui est comprise entre environ 37 et environ 50 degrés Celsius.

De cette manière, en contrôlant la température d'une des surfaces conductrices, on peut, grâce aux particules et/ou fibres conductrices comprises dans le matériau composite, contrôler la forme de l'aiguille et la modifier rapidement à tout instant.

Avantageusement, $Ts_1 = Ts_2 = TS$.

Préférentiellement, la surface de l'aiguille pourra être traitée pour obtenir un coefficient de frottement faible et ainsi faciliter la pénétration dans les tissus.

Le matériau composite utilisé possède une conductivité thermique suffisante pour que le changement de température s'effectue dans toute l'aiguille dans un délai maximum d'environ 15 secondes, lorsque l'on chauffe ou que l'on refroidit une extrémité de l'aiguille par sa surface conductrice.

Préférentiellement, ce délai sera au maximum d'environ 10 secondes.

De manière encore plus préférentielle, ce délai sera au maximum d'environ 5 secondes.

A l'une des extrémités de l'aiguille est préférentiellement attaché un fil de suture chirurgical classiquement utilisé en chirurgie.

Avantageusement, cette aiguille peut être utilisée comme une aiguille diathermique.

Avantageusement, l'aiguille selon la présente invention peut aussi être utilisée par d'autres voies d'accès que l'endoscopie thérapeutique souple telles que par exemple à travers les trocarts pédiatriques.

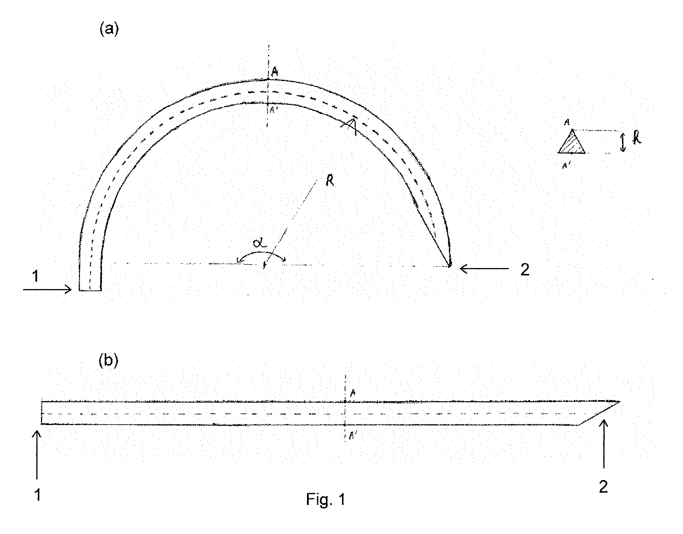

Les figures 1a et 1b présentent les deux formes de l'aiguille - forme courbe et forme droite - selon un premier mode préférentiel de réalisation de la présente invention.

Dans ce mode de réalisation préférentielle, l'extrémité 1 de l'aiguille se prolonge par un segment rectiligne.

La température de transition entre ces formes est d'environ 40°C.

Le rayon R de la forme courbe de l'aiguille est d'environ 12.5 mm.

L'angle α formé par les rayons joignant les deux extrémités de la partie courbe de cette aiguille est d'environ 180°.

La section de l'aiguille est un triangle équilatéral de hauteur h égale à environ 1.6 mm.

L'aiguille est constituée de fibres conductrices situées dans un matériau polymère à mémoire de forme.

Les fibres conductrices sont liées aux deux surfaces conductrices situées aux deux extrémités de l'aiguille.

A son extrémité 2, l'aiguille s'amincit de manière à former un biseau

A son extrémité 1 est attaché un fil chirurgical.

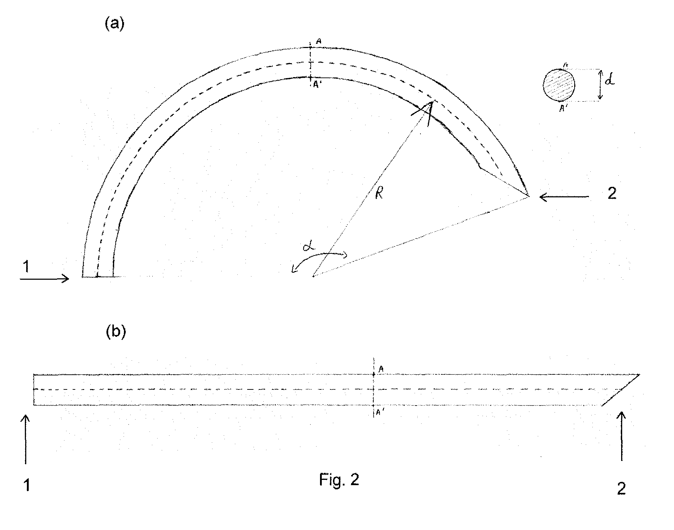

Les figures 2a et 2b présentent les deux formes de l'aiguille - forme courbe et forme droite - selon un second mode préférentiel de réalisation de la présente invention.

Dans ce mode de réalisation préférentielle, l'extrémité 1 de l'aiguille ne se prolonge pas par un segment rectiligne.

L'extrémité 2 de l'aiguille est affûtée en biseau.

La température de transition entre ces formes est d'environ 45°C.

Le rayon R de la forme courbe de l'aiguille est d'environ 20 mm.

L'angle α formé par les rayons joignant les deux extrémités de la partie courbe de cette aiguille est d'environ 160°.

La section de l'aiguille est un cercle dont le diamètre d est d'environ 2.8 mm.

L'aiguille est constituée d'un matériau polymère à mémoire de forme contenant une répartition de particules et/ou de fibres conductrices.

A son extrémité 2, l'aiguille s'amincit de manière à former une pointe.

A son extrémité 1 est attaché un fil chirurgical.

**Exemples**

1. Plicature gastrique transmurale

L'aiguille présentée aux figures la et 1b a été utilisée pour réaliser une plicature gastrique transmurale sur un estomac de porc *ex vivo.*

Ladite aiguille étant insérée dans l'endoscope, sous forme droite, l'expérimentateur la pousse à travers la paroi située quelques centimètres sous la ligne Z. Une fois insérée aux deux tiers au travers de la paroi gastrique, elle est chauffée afin de se courber à 180°.

L'aiguille est alors retirée à l'aide de l'endoscope et son extrémité 2 a été saisie par une pince introduite dans le deuxième canal opérateur.

Après réalisation de ce premier passage, l'aiguille est refroidie, retournée, réinsérée au travers de la paroi de l'estomac, et à nouveau chauffée pour retrouver sa courbe à 180°.

Cette manoeuvre est réalisée trois fois de manière consécutive afin d'obtenir une triple plicature de l'estomac.

L'aiguille a alors été refroidie et récupérée droite au travers du canal opérateur de l'endoscope.

Une suture a été effectuée sur les 2 brins du fil attaché à l'aiguille. 2. Suture gastrique en surjet

L'aiguille présentée aux figures 1a et 1b a été utilisée pour réaliser une plicature gastrique en surjet sur un estomac de porc *ex vivo.*

Dans un premier temps, ladite aiguille est insérée dans sa forme droite dans la face postérieure de l'antre.

Elle est ensuite chauffée pour se courber à 180°, récupérée avec le deuxième canal opérateur de l'endoscope, après avoir retraversé, de l'extérieur vers l'intérieur, la paroi gastrique.

L'aiguille et son fil sont ensuite ramenés sur une longueur d'environ 25 cm dans la cavité gastrique et les 2 brins émergeant de la paroi gastrique sont solidarisés via le deuxième canal opérateur.

L'aiguille est alors refroidie, réinsérée dans la paroi antérieure de l'estomac, réchauffée, récupérée dans la cavité gastrique, refroidie, réinsérée dans la paroi postérieure de l'estomac, réchauffée et récupérée dans la cavité gastrique, et ainsi de suite pour obtenir un surjet à cinq ou six sutures, permettant d'apposer la face postérieure de l'estomac à sa face antérieure.

A la fin du surjet, l'aiguille est récupérée dans la cavité gastrique, courbée sous l'effet de la chaleur et insérée entre les mailles du dernier surjet, afin de réaliser un double noeud chirurgical.

3. NOTES

Dans un modèle de NOTES (Natural Orifice Transluminal Endoscopic Surgery), sur animal vivant, une perforation iatrogène de la cavité gastrique est réalisée à l'aide d'une aiguille diathermique et d'une dilatation au ballon d'environ 18 mm.

L'endoscope est inséré dans le péritoine et, au retrait de celui-ci, l'aiguille, décrite dans les figures 1a et 1b, est insérée dans sa forme droite dans l'endoscope, la paroi gastrique ayant été ponctionnée.

A ce moment, l'aiguille est chauffée afin de se courber à 180°.

Son extrémité ponctionne alors la paroi externe contralatérale de l'estomac, par rapport à l'orifice.

L'aiguille est récupérée dans la cavité gastrique et est ensuite refroidie.

Une nouvelle ponction est effectuée, décalée de 90° par rapport à la précédente, avec l'aiguille dans sa forme droite.

L'aiguille est alors réchauffée, récupérée dans la cavité gastrique et, sur les 2 brins résultant de ce point en croix, une suture est appliquée.

4. Suture en endoscopie rigide

L'aiguille présentée aux figures la et 1b est insérée dans sa forme droite, dans le péritoine d'un animal vivant, au travers d'un trocart pédiatrique. Une fois dans le péritoine, elle est chauffée afin de se courber à 180°. **[0110]** Nous disposons alors d'une aiguille courbe dans le péritoine que nous pouvons utiliser comme une aiguille classique de chirurgie pour effectuer une suture. Une fois la suture effectuée, l'aiguille est refroidie afin de retrouver sa forme droite. Elle est ainsi retirée de la cavité péritonéale au travers du trocart pédiatrique.

**Revendications**

1. Aiguille chirurgicale à mémoire de forme comprenant un corps d'aiguille:

   - ayant deux extrémités (1,2), une extrémité proximale (1) et une extrémité distale (2),
   - ledit corps présentant au moins deux formes stables différentes, une première forme stable à une première gamme de température (T1) et une deuxième forme stable à une deuxième gamme de température (T2), la première gamme de température étant strictement inférieure à la deuxième gamme de température, **caractérisée en ce que :**
   - le matériau constituant le corps de l'aiguille comprend au moins un matériau composite à mémoire de forme et au moins un matériau conducteur thermique et/ou électrique,
   - la totalité du corps de l'aiguille est apte à être chauffée en appliquant une source de chaleur et/ou de courant, la variation de température induite par la source de chaleur et/ou de courant dans l'aiguille permettant le passage de la première forme stable à la deuxième forme stable.

2. Aiguille chirurgicale selon la revendication 1 **caractérisée en ce que** la totalité du corps de l'aiguille est apte à être refroidie en appliquant une source de froid, la variation de température induite dans l'aiguille permettant le passage de la deuxième forme stable à la deuxième gamme de température (T2) à une troisième forme stable à une troisième gamme de température (T3).

3. Aiguille chirurgicale selon la revendication 2 **caractérisée en ce que** la troisième forme stable correspond à la première forme stable.

4. Aiguille chirurgicale selon l'une quelconque des revendications précédentes, **caractérisée en ce** au moins une des extrémités de ladite aiguille est réalisée en un matériau conducteur thermique et/ou électrique qui est différent ou identique du matériau conducteur thermique et/ou électrique constituant le corps de l'aiguille, ladite extrémité étant en contact direct avec ledit matériau conducteur contenu dans le reste de l'aiguille.

5. Aiguille chirurgicale à mémoire de forme selon une quelconque des revendications précédentes, **caractérisée en ce que** la forme de l'aiguille à une première gamme de température (T1) est rectiligne.

6. Aiguille chirurgicale à mémoire de forme selon une quelconque des revendications précédentes, **caractérisée en ce que** la forme de l'aiguille à une deuxième gamme de température (T2) est courbe.

7. Aiguille chirurgicale à mémoire de forme selon une quelconque des revendications précédentes, **caractérisée en ce que** l'extrémité distale(2) de l'aiguille est affûtée.

8. Aiguille chirurgicale à mémoire de forme selon une quelconque des revendications précédentes, **caractérisée en ce que** l'extrémité proximale(1) de l'aiguille accueille du fil chirurgical.

9. Dispositif permettant la suture d'une muqueuse située à l'intérieur du corps humain **caractérisé en ce qu'**il comprend une aiguille chirurgicale selon l'une quelconque des revendications précédentes et une source de chaleur et/ou de courant en contact direct avec l'une des extrémités (1,2) et en particulier l'extrémité proximale (1) de l'aiguille.

10. Dispositif selon la revendication 9, **caractérisé en ce qu'**il comprend une source de froid en contact direct avec l'une des extrémités (1,2) et en particulier l'extrémité proximale (1) de l'aiguille.

11. Utilisation de l'aiguille selon l'une quelconque des revendications 1 à 8, ou du dispositif selon les revendications 9 ou 10 pour des actes thérapeutiques et en particulier des sutures à effectuer par voie endoscopique.

(a)

(b)

Fig. 1

(a)

(b)

Fig. 2

Europäisches
Patentamt

European
Patent Office

Office européen
des brevets

**RAPPORT PARTIEL
DE RECHERCHE EUROPEENNE**
qui selon la règle 63 de la Convention sur le brevet
européen est consideré, aux fins de la procédure ultérieure,
comme le rapport de la recherche européenne

Numéro de la demande

EP 08 16 9414

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | WO 95/08296 A (KIM YOUNG KON [KR]; DOO JAE KYUN [US]) 30 mars 1995 (1995-03-30) <br> * page 2, ligne 20 - page 3, ligne 1 * <br> * page 3, ligne 40 - page 4, ligne 6 * <br> * page 5, ligne 6 - page 6, ligne 38 * <br> * figures 2A,2B * <br> ----- | 1-10 | INV. <br> A61B17/06 <br><br> ADD. <br> A61B17/00 <br> A61B18/00 |
| X | DE 10 2004 024188 A1 (SCHNEPP-PESCH WOLFRAM [GB]) 16 février 2006 (2006-02-16) <br> * alinéas [0008], [0054] - [0056] * <br> * figures 5a-c,10a-e * <br> ----- | 1-8 | |
| X | WO 2005/077279 A (ECLECTIC GREY MATER DESIGNS LL [US]; MEYER MATTHEW EARL [US]) 25 août 2005 (2005-08-25) <br> * alinéas [0015], [0016], [0022], [0023], [0036], [0047], [0048], [0055], [0201], [0204] - [0207] * <br> * figures 1,2A,2B,2.2,2.2A-C * <br> ----- <br> -/-- | 1-4,7-10 | |

**DOMAINES TECHNIQUES
RECHERCHES (IPC)**

A61B

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| Munich | 3 avril 2009 | Maier, Christian |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04E08)

Europäisches
Patentamt

European
Patent Office

Office européen
des brevets

**RAPPORT PARTIEL
DE RECHERCHE EUROPEENNE**

Numero de la demande

EP 08 16 9414

| | DOCUMENTS CONSIDERES COMME PERTINENTS | | CLASSEMENT DE LA DEMANDE (IPC) |
|---|---|---|---|
| Catégorie | Citation du document avec indication, en cas de besoin, des parties pertinentes | Revendication concernée | |
| X,D<br><br>Y | EP 0 529 675 A (ETHICON INC [US])<br>3 mars 1993 (1993-03-03)<br>* colonne 5, ligne 32 - ligne 44 *<br>* colonne 6, ligne 29 - colonne 7, ligne 14 *<br>* figures 1-5 *<br>----- | 1,4-10<br><br>2,3 | |
| Y | US 5 624 508 A (FLOMENBLIT JOSEF [IL] ET AL) 29 avril 1997 (1997-04-29)<br>* colonne 1, ligne 56 - ligne 65 *<br>* colonne 6, ligne 22 - ligne 25 *<br>* revendication 1 *<br>----- | 2,3 | |
| | | | DOMAINES TECHNIQUES RECHERCHES (IPC) |

EPO FORM 1503 03.82 (P04C11)

Europäisches
Patentamt

European
Patent Office

Office européen
des brevets

**RECHERCHE INCOMPLETE
FEUILLE SUPPLEMENTAIRE C**

Revendications n'ayant pas fait l'objet de recherches:
    11

Raison pour la limitation de la recherche (invention(s) non brevetable(s)):

Article 53 (c) CBE - Méthode de traitement chirurgical du corps humain ou animal
Article 53 (c) CBE - Méthode de traitement thérapeutique du corps humain ou animal

**ANNEXE AU RAPPORT DE RECHERCHE EUROPEENNE**
**RELATIF A LA DEMANDE DE BREVET EUROPEEN NO.**

EP 08 16 9414

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-04-2009

| Document brevet cité au rapport de recherche | | Date de publication | Membre(s) de la famille de brevet(s) | | Date de publication |
|---|---|---|---|---|---|
| WO 9508296 | A | 30-03-1995 | AUCUN | | |
| DE 102004024188 | A1 | 16-02-2006 | AUCUN | | |
| WO 2005077279 | A | 25-08-2005 | US | 2007135838 A1 | 14-06-2007 |
| EP 0529675 | A | 03-03-1993 | AU | 667673 B2 | 04-04-1996 |
| | | | AU | 2126792 A | 22-04-1993 |
| | | | BR | 9203381 A | 06-04-1993 |
| | | | CA | 2077063 A1 | 01-03-1993 |
| | | | DE | 69208182 D1 | 21-03-1996 |
| | | | DE | 69208182 T2 | 05-09-1996 |
| | | | EG | 19806 A | 29-02-1996 |
| | | | ES | 2083039 T3 | 01-04-1996 |
| | | | GR | 92100362 A | 07-06-1993 |
| | | | IL | 102861 A | 14-05-1996 |
| | | | JP | 3700089 B2 | 28-09-2005 |
| | | | JP | 5200036 A | 10-08-1993 |
| | | | TR | 26729 A | 15-05-1995 |
| | | | US | 5219358 A | 15-06-1993 |
| US 5624508 | A | 29-04-1997 | AU | 2402397 A | 24-11-1998 |
| | | | WO | 9849363 A1 | 05-11-1998 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9508296 A

- EP 0529675 A